# EUROPEAN PATENT SPECIFICATION

(11) **EP 4 291 302 B1**
(45) Date of publication and mention of the grant of the patent: **14.01.2026**
(21) Application number: 22712795.8
(22) Date of filing: 11.02.2022
(51) Int. Cl.: A61N 2/00, A61N 2/02

(54) **MAGNETIC STIMULATION COIL**
MAGNETISCHE STIMULATIONSSPULE
BOBINE DE STIMULATION MAGNÉTIQUE

(30) Priority: 15.02.2021 CZ 20210067
(43) Date of publication of application: 20.12.2023
(73) Proprietor: DEYMED Diagnostic s.r.o., 549 31 Hronov (CZ)
(72) Inventor: MORAVEC, Miroslav, 54931 Hronov (CZ)
(74) Representative: Pavlica, Tomas
(86) International application number: PCT/CZ2022/000009
(87) International publication number: WO 2022/171218

(56) References cited:
- WO-A1-2019/150378
- WO-A1-2019/161407
- WO-A2-02/32504

## Description

### Field of the Art

The invention relates to a magnetic stimulation coil which can be used as a coil for magnetic stimulation in neurophysiology, and particularly preferably for repetitive transcranial magnetic stimulation (rTMS) in psychiatry.

### Prior Art

In neurophysiology, the magnetic stimulation is an established term used generally for stimulation of any nerve structures, including those of the brain, mainly for diagnostic and research purposes, by means of an induced electrical voltage, the voltage is induced by a rapid change of a strong magnetic induction over time. In psychiatry, this term is narrowed to the stimulation of the brain for therapeutic purposes, especially to treat depressions and other illnesses.

The induced electrical voltage must have a sufficient intensity (of the order of tens of V/m and last long enough, in the order of tens to hundreds of µs) to depolarize the cell membranes of the nerve cells, and thus to induce some electrophysiological response. The magnetic stimulation is used both for diagnostic purposes in neurophysiology and, more recently, also for therapeutic purposes in psychiatry.

For the purposes of repeated magnetic stimulation of the brain with a repetitive frequency in the order of units up to tens of Hz, the term repetitive transcranial magnetic stimulation (rTMS) was introduced in the psychiatry due to use of the repeated stimulation in variously designed stimulation protocols.

Until a few years ago, standardly, low-frequency protocols with a stimulation level of 80% of the motoric threshold with a frequency in the order of units of Hz were used for the purposes of the repetitive transcranial magnetic stimulation (rTMS). In a simplified way, the motoric threshold can be understood as such intensity of the magnetic stimulation, at which threshold irritation of the motoric cortex takes place, what is objectively measured by surface electrodes sensing the EMG signal from a muscle, which is innervated by the relevant motoric cortex region, or visually by observing the induced movement.

Due to the fact that even with increasing the overall intensity of the stimulation, the benefit against side effects is still increasing, protocols stimulating up to 130% of the so-called motoric threshold are currently being used to treat depression and other diseases. Also, this makes possible to shorten the duration of therapy, and thus it also leads to a greater spread of such intensive protocols in clinical practice.

Nevertheless, it turns out that the limiting factor of this trend are not so much the ever-increasing demands on sufficient power of the magnetic stimulators and on the cooling of the stimulation coils, but it is the patient's willingness to accept the side effects of the stimulation in the form of unpleasant, and in cases of the very intense protocols even painful, stimulation of the surrounding head muscles.

In terms of the degree of implementation, the current state of development of the stimulation coils can be divided into three basic regions:
- Commercially produced stimulation coils used in clinical practice;
- Experimental stimulation coils for research purposes;
- Physical models of the stimulation coils;

At present, the octal stimulation coils consisting of two circular windings placed side by side are mainly used for the purposes of the rTMS therapy because to stimulate the appropriate cortex structures it is necessary to make the vector of the induced electrical voltage (and thus also of the current) to be parallel to the surface of the skull. This is difficult to ensure with the conventional circular stimulation coils.

The up to date design solution of the commercially produced stimulation coils uses an overlap of the originally octal shape windings, what partially reduces the adverse effects of the lateral lobes of the magnetic field on the surrounding muscles. Focusing of the magnetic field into the regions of the motoric cortex is performed by partial tilting of both parts of the coils, what is disclosed at best in the document Electric field properties of two commercial figure-8 coils in TMS: calculation of focality and efficiency, Clinical Neurophysiology 115 1697-1708 (2004), by Axel Thielscher and Thomas Kammer, and in the document "Safe exposure distances for transcranial magnetic stimulation based on computer simulations, Postgraduate Program in Metrology, Pontifícia Universidade Católica do Rio de Janeiro, Rio de Janeiro, Brazil", by lam Palatnik de Sousa, Carlos R. H. Barbosa, and Elisabeth Costa Monteiro (2018).

Also, stimulation coils comprising an iron core are used to focus the magnetic field, which coils are limited by the magnitude of the magnetic induction to about 2 Tesla, because at higher intensities, the magnetic material of the coil core is saturated.

Other stimulating coils are presented in the document of the authors Samuel Zibman, Gaby S. Pell, Noam Barnea-Ygael, Yiftach Roth, Abraham Zangen (2019) "Application of transcranial magnetic stimulation for major depression: Coil design and neuroanatomical variability considerations, European Neuropsychopharmacology 1 - 16". These stimulation coils are used for the purposes of the deep brain stimulation. However, the deep brain stimulation is applied for different therapeutic uses, and also, these coils do not address stimulation of the head subordinate muscles.

In the documents "Quantitative assessment of the focality of a double-D coil in the human brain, Conference: 2017 XXXIInd General Assembly and Scientific Symposium of the International Union of Radio Science (2017)" by authors Yuta Kawasaki, Koichi Hosomi, Keita Yamamoto, Shintaro Hara, Yusuke Abe, Youichi Saitoh, and Masaki Sekino, and "Novel coil designs for different neurological disorders in transcranial magnetic stimulation (2019)", Graduate Theses and Dissertations. 17547; https://lib.dr.iastate.edu/etd/17547 by author Rastogi Priyam, experimental stimulation coils used to focusation of magnetic field are described. These stimulation coils are comprised of 4 circular parts, or the stimulation coil is shaped to form a space tightly enclosing the head. However, with regard to these shapes of the stimulating coils, a solution, which is necessary to achieve sufficient stimulation intensity in the desired region while maintaining a low intensity outside this region, is not described.

Several studies, mainly from the university environment, deal with the research of the possibilities of the magnetic field focusing in the stimulation coils, i.e. the document: "Minimum-energy Coils for Transcranial Magnetic Stimulation: Application to Focal Stimulation". Brain Stimulation, 8 (1), 124-134. https://doi.org/10.1016/j.brs.2014.10.002 by authors L. M. Koponen, J. O., Nieminen, and R. J. Ilmoniemi, further the document "Advances in transcranial magnetic stimulation technology. Brain Stimulation, (2015), June 165-189 by Angel V. Peterchev, Zhi-De Deng, and Stefan Goetz, the document "Coil optimization for transcranial magnetic stimulation in realistic head geometry, Brain Stimulation 10 795-805" (2017) by L. M. Koponen et al, the document "Multi-locus transcranial magnetic stimulation theory and implementation, Brain Stimulation 11 849-855" (2018) also by L. M. Koponen et al. and the document "A 3-axis coil design for multichannel TMS arrays, Neurolmage 224" (2021) by L. I. Navarro de Lara, M. Daneshzand, A. Mascarenas et al. However, the models described therein currently work with thin electrical conductors, whose cross-section does not limit the resulting shape of the stimulation coil. A disadvantage of such coils is that they cannot be used for the above-threshold repetitive magnetic stimulation, due to both the very rapid overheating of the conductor and also due to the need to use a magnetic stimulator with a power output that could not be supplied by a common electric connection. Already the currently used magnetic stimulators require electric power input, which is at the limit of possibilities of the electric supply network, because stimulation of the biological tissues by the electromagnetic induction is very energy inefficient.

Further, documents WO2019150378 and US2018/280711 show TMS coils of a butterfly shape.

Further, experience shows that the limited electric conductivity of copper at room temperature is the limiting factor determining the construction of shape of the stimulation coil, provided that the coil can be properly cooled during the longer stimulation protocols.

### Summary of the Invention

The above-mentioned drawbacks, including the deficiency in the form of the undesired stimulation of the insignificant structures located in the immediate vicinity of the stimulation site, are overcome, or at least largely suppressed by a stimulation coil for the magnetic stimulation according to this invention.

**Stimulation coil consisting of two symmetrical halves, a left half and a right half. Both halves are arranged next to each other and in side-by-side relation. The winding of each half is formed by a set of turns made of a conductor.**

The stimulation coil according to the present invention is based on the idea of dividing it, more precisely it's windings, into two basic regions: a central region and refluent region. This division was carried out to make it easier to optimize the individual parts of said coil, since optimizing of the entire winding length would be a much more complex problem.

In comparison to the known stimulation coils, where the entire length of the conductor contributes proportionally in its whole length to the magnetic field induction, owing to the division of the stimulation coil into the central region and the refluent region, it is possible to approach each of the two regions separately and to optimize the required characteristics involved in inducing of a magnetic stimulus. The central region is formed by individual winding layers, hereinafter referred to as tiers, of conductor turns, which are arranged here as close to each other as possible to induce the strongest possible magnetic field in the focus situated under its centre, wherein, particularly preferably the turns in the central region are arranged substantially linearly, i.e. here, the turns form parallel lines in at least one third of the length, whereas, the refluent region of each half of the coil is optimized both to achieve the lowest possible refluent contribution to the magnetic induction, to avoid reducing of the effect of the central region, and to avoid formation of regions of strong stimulation outside the intended focus to avoid formation of excessive and unpleasant stimulation of the head muscles.

The objective of the design of the stimulation coil according to the present invention is to situate the focus of the magnetic field, induced by this stimulation coil, so that it is located at a depth of about 2 to 3 cm below the side **of the stimulation coil intended for an application of the magnetic stimulation.**

Thus, in the central region, the conductor turns in individual tiers are arranged at least in a part of the central region linearly, i.e. in parallel to each another and closely one on another, in order to ensure their greatest possible density and efficiency, wherein, preferably, this linear arrangement is provided at least in the length of about 1/3 of the longer diameter of the ellipse, see further below, more preferably in its entire length. The conductor turns in the refluent region are arranged substantially as elliptical shapes starting from the central region, wherein, the turns are having a ratio of the semi-axes of the ellipse, they form essentially, i.e. without any ellipse "deformation" caused by the linear course of the turns in the central region., in which they already do not form this ellipse, in the range of 0.3 to 1, more preferably 0.5 to 0.8, and most preferably about 0.6 to 0.7, to achieve the highest possible stimulation intensity in the intended focus. The conductors in the central and in the refluent regions are arranged at least as three tiers, which tiers are placed one on top of the other, wherein the end of the last conductor, i.e. of the outer turn in one tier of one half of the winding, is connected with the conductor of the first, i.e. the inner turn in the following higher tier of the same winding half. **All the tiers of each half of said winding are connected in series and have the same direction of the conductor rotation, wherein said direction of conductor rotation is opposite in each half of the winding to provide in the central region the same direction of current passing through the turns of the winding in both halves of the stimulation coil.**

Although three tiers are enough for a sufficient effect, more preferably, the stimulation coil comprises rather four or five, or even more tiers. Division of the coil turns into at least three tiers is also advantageous due to more even distribution of the turns in the refluent region, the consequence of which is an increased ratio between the magnetic field strength in the focus region and at the edges of the stimulation coil. In order that the tiers, which are located at a greater distance from a surface plane would have approximately identically placed their own stimulation focus, gradual widening of the winding dimensions of the individual tier takes place in the refluent region.

Structure of the stimulation coil according to the invention is such that it is formed of tiers of turns forming halves of windings of proportionally increasing size in the direction from the application side, i.e. so as their distance from the central stimulus point increases, wherein, the ratio of the magnetic field magnitude in the intended focus of the stimulation coil to the magnitude of the magnetic field along the edges of the coil increases, and so it ensures directing of the magnetic field towards this focus.

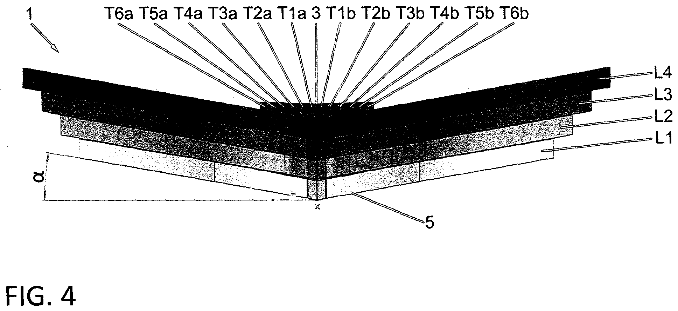

According to a particularly preferred embodiment of the stimulation coil according to the invention, widening of the individual turns in each further tier in the region of the vertices of the longer half-axes of the ellipses in the direction from the application side is provided so that the outside points of the circumferences in their succession from the centre of the respective turns of individual tiers form a line forming an angle approximately 45 degrees with the plane of stimulation so as it is evident for example in Fig. 4 for the outer turns of individual tiers of both halves of the windings of the stimulation coil. Such a line can be intersected also through the outside points of the circumferences of the other turns in the individual tiers, but they are not visible in Fig. 4.

To prevent such spreading of the magnetic field that would form significant regions with a strong magnetic field situated outside the intended region, the conductor turns in the refluent region within the individual tiers from their point of their separation from the central region are made loose by arranging them with spacing from each other, where this spacing is the largest in the area of the longer half-axis of the ellipse, in which the conductor turns are situated, and the spacing is equal approximately at least to the size of a half of the conductor dimension, from which conductor the turns are formed, preferably of more than one conductor dimension in the given direction, particularly preferably in the range from one to three conductor dimensions, wherein as the conductor dimension is understood for the purposes of this application its dimension in the spreading direction of the turns of one tier.

According to another preferred embodiment the stimulation coil according to the invention has its two winding halves tilted away from the application side at an angle of tilting about 5 to 20 degrees, preferably about 10 degrees from the surface plane.

The angle of diverting is chosen as a compromise so that in case of a too high angle there is not any decrease of stimulation intensity in the focus and that in case of a too low angle there is not shown any negative influence on increasing of the stimulation intensity in the lateral regions.

The above described tilting within the specified diverting angle range will significantly reduce the undesired effects of the lateral lobes, what would otherwise result in undesirable unpleasant stimulation of the head muscles.

According to a particularly advantageous embodiment of the stimulation coil according to this invention, the turns in the central region on the application side are particularly preferably adapted into a substantially spherical depression having the maximum depth in the range from 0.5 cm to 1.5 cm, particularly preferably about 1 cm, to allow the possibility to attach the central region on the application side to any almost arbitrarily individually shaped skull so that it is prevented that the scalp does not abut against the surface of the stimulation coil on the application side, in such a way that the contributions of the windings of both halves of the coils is as high as possible in the focus point. An advantage of this design in comparison to the flat design of the central region is that it produces about 10 to 15 percent increase in the magnitude of the stimulation intensity.

### Description of Drawings

The invention will be more readily understood from the following examples of its embodiments and from the accompanying drawings, in which:
Fig. 1 shows a top view of a preferred embodiment of a stimulation coil according to the invention provided with three tiers;
Fig. 2 is a bottom view of the stimulation coil of Fig. 1;
Fig. 3 is a perspective view of a half of windings of a stimulation coil, which is similar to that of Fig. 1, but which is provided with four tiers;
Fig. 4 is a side view of a stimulation coil with four tiers;
Fig. 5 is a top view of the bottom tier of the stimulation coil of Fig. 1.

### Examples of Embodiments of the Invention

For a better understanding of the stimulation coil according to the invention, examples of its possible implementation will be described below.

The present invention is defined in the following claims. Other embodiments, examples, items etc. are not a part of the invention.

For illustrative purposes, some elements may be enlarged in size in the drawings and may not be drawn to scale. Dimensions and relative dimensions do not correspond to actual reductions. The terms, such as the right, the left refer exclusively to the illustrations of the stimulation coil as shown in the drawings, and such terms serve only for the purpose to distinguish the shown symmetrical parts of the stimulation coil according to the invention. The designations bottom and upper refer to the illustrations of the stimulation coil, wherein the bottom side is considered to be the application side, which is situated at the bottom in the figures. Thus, it is clear that for example in reality the right half may be on the left side, and vice versa, as well as, depending on the stimulation performed, the bottom, i.e. application side may be upper one, and the upper one may by at the bottom.

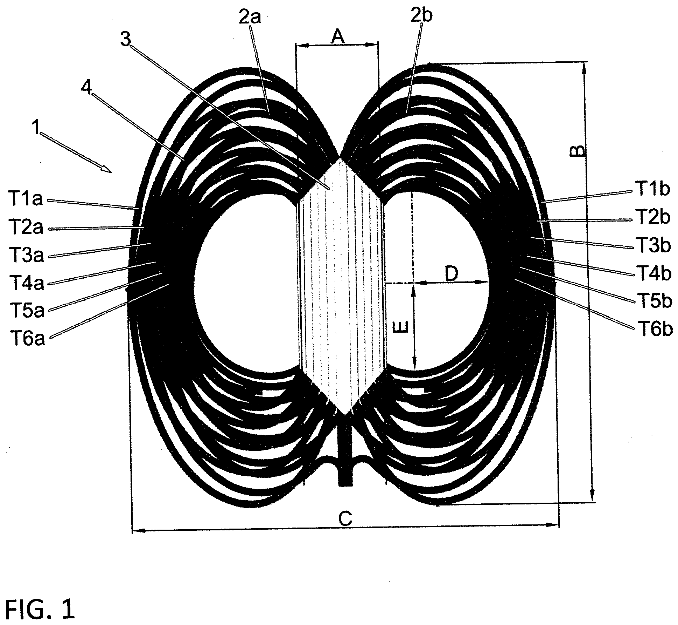

Fig. 1 shows schematically a top view of one possible embodiment of a stimulation coil 1 according to this invention. In this figure, for clearness, the central region 3 of the stimulation coil, formed by the respective turn sections T1a to T6a and T1b to T6b of both winding halves 2a, 2b, is shown in light colour, while the return region 4 formed by the remaining turn sections T1 to T6 of both winding halves 2a, 2b is depicted in dark colour. However, these colour differences are created only for the purpose to differentiate the two regions more clearly.

The turns T1a to T6a and T1b to T6b of the individual tiers L1 to Ln of the two winding halves 2a, 2b of the stimulation coil 1 are made of a conductor in the illustrated embodiment, which in this embodiment is a cable of a litz wire type, hereinafter referred to as litz wire cable, of rectangular cross-section profile. The number "n" corresponds to the number of tiers, so that for a stimulation coil with three tiers the coil has tiers L1 to L3, for a stimulation coil with four tiers, the coil has tiers L1 to L4, etc.

The rectangular profile is particularly advantageous because it allows the most tight arrangement of the coil turns in the central region.

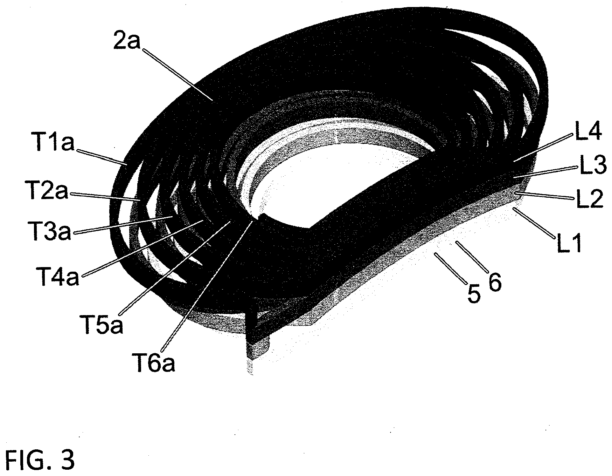

In the given embodiment, the litz wire cable of the has dimensions 4 mm x 2 mm and consists of 160 from each other insulated conductors with a diameter of 0.2 mm. In this exemplary embodiment, said litz wire cable is used both to prevent the formation of eddy currents in the conductor itself and to prevent the negative influence of the differential loops in the curvature of the turns in a given half of the winding 2a, 2b. The turns T1a to T6a and T1b to T6b are made on the individual tiers L1 to Ln of the stimulation coil 1 in such a way that the larger dimension of the rectangular cross-section of the litz wire cable is oriented in the vertical direction, what can be seen in Fig. 3.

In this embodiment, the stimulation coil 1 is made with an air core, as the coil is intended to be used both for stimulation with a biphasic course of the stimulation intensity of the magnetic induction and for monophasic stimulation, when using a higher intensity of magnetic induction is necessary to bring about the same electrophysiological response. A stimulation coil with a ferromagnetic core may no longer be able to induce such a high magnetic induction. A secondary reason is the possibility to use the stimulation coil in a magnetic resonance environment.

Although the design of the air core comprising stimulation coil 1 appears to be more advantageous for the reasons described above, this does not mean that the stimulation coil 1 is limited only to such an embodiment.

For example, the use of a stimulation coil with a ferromagnetic core for clinical therapeutic purposes and the like is contemplated.

However, it is important to realize that the specified dimensions of the litz wire cable used serve only an example, and not as a limitation. Likewise, the use of the litz wire cable itself is intended only as a particularly advantageous realization of the stimulation coil according to the invention, but it is certainly not intended to be the only possible embodiment of the conductor.

The expert is able to suggest also other possible embodiments.

It is important to realize that both the conductor type used for the turns of the stimulation coil 1 and the dimensions of the litz wire cable used, are certainly not intended as those limiting the invention only to the given example.

Further, it is important to emphasize that in the above-mentioned examples of embodiment, all turns in individual tiers of each half of the stimulating coil are made of a single conductor and that the beginning and ending parts of the turns in individual tiers are joined so as to form the desired one half of a winding from a group of turns of a conductor in individual tiers.

However, it is obvious to the person skilled in the art that other embodiment is also possible, for example to form each half of the winding from one conductor, i.e. to wind the turns of the individual tiers gradually, so that it will not be necessary to interconnect them. In the same way, it is possible to design also the turns in individual tiers from several separate conductors, which conductors are interconnected, and the like.

The stimulation coil of the exemplary embodiment in Figures s 1 and 2 is provided with a winding composed totally of three tiers L1 to L3 of turns and exhibits the following dimensions: the height B of the stimulation coil 1 is 125 mm, the width C of the stimulation coil 1, i. e. the width of both halves 2a, 2b of the winding of the stimulation coil 1 next to each other, and this including the central region 3, is 124 mm, the width A of the central region 3 of the stimulation coil 1, in which the turns of the individual tiers L1 to L3 of both winding halves are arranged as close together as possible, is 24 mm, the length D represents the length of the shorter half-axis of the ellipse of the internal turn T5b of the refluent region 4 of the bottom tier L1 of the stimulation coil 1, i.e. of the turn which is as close as possible to the centre of the stimulation coil 1, is 20 mm, and the length E of the longer half-axis of the ellipse of the mentioned internal turn T5b of the refluent region 4 is approx. 23 mm.

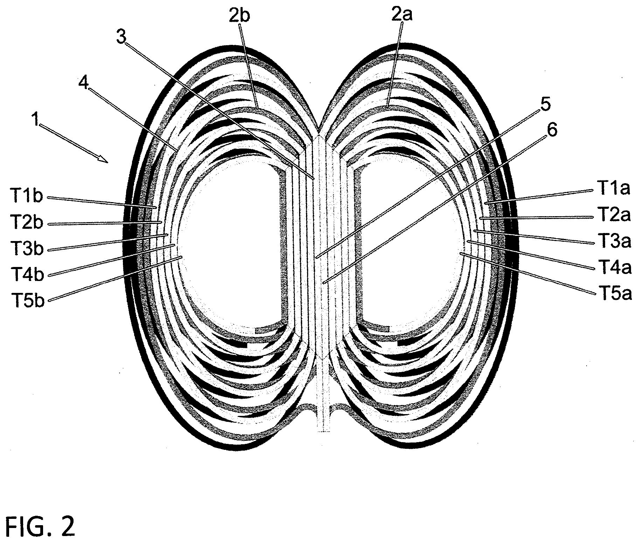

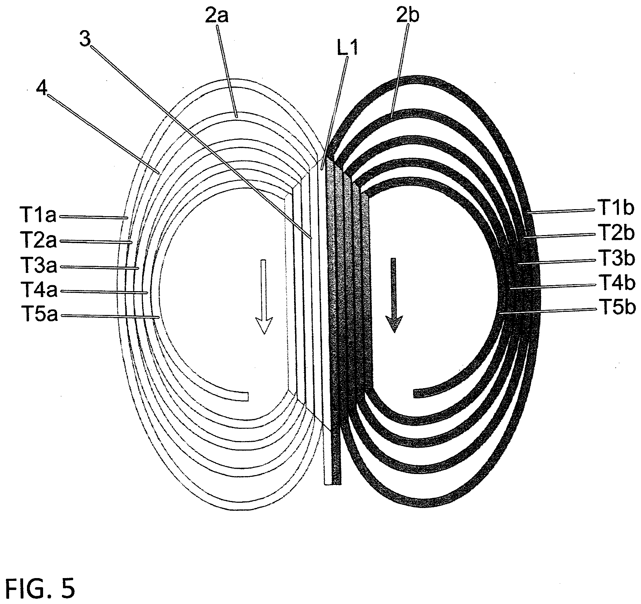

In the bottom first tier L1 of each of the both halves 2a, 2b of the winding of the stimulation coil 1, i.e. in the tier whose bottom side forms the application side 5, five turns T1a to T5a and T1b to T5b of the conductor are arranged which conductor is the above-mentioned litz wire cable. The turns T1a to T5a and T1b to T5b of the conductor are arranged as close to each other as possible in the central region 3, and they are substantially parallel to one another in the entire length as far as it is possible, whereas in the refluent region 4 they are arranged substantially in the shape of ellipse, wherein, at least in the region of both mutually opposite more distant vertices of the ellipse, i.e. in the region of the vertices of the longer half-axis of said ellipse, the conductors are spaced from each other with a gap making at least a half the smallest of dimensions of the conductor cross-section, whereby a loosening between the individual turns is formed, as it is apparent in Fig. 2 or Fig. 5.

In the other tiers of the stimulation coil 1, i.e. in the tiers L2 and L3, always six turns T1a to T6a and T1b to T6b of the conductor are arranged in one tier, wherein, these turns T1a to T6a and T1b to T6b are arranged as close to each other as possible in the central region, and they are linear, the same goes parts of the turns T1a to T5a and T1b to T5b of the tier L1, whereas, in the refluent region they are loose and they widen upwards, as it can be seen in Fig. 4.

The spacing between individual turns is here up to the distance of three wire dimensions. The respective turns T1a to T6a and T1b to T6b in the refluent region in the individual tiers L1 to L3 are made with an increasing ratio of the radii of the ellipse axes. The axes radii T1a to T5a and T1b to T5b, resp. T1a to T6a and T1b to T6b in the individual tiers L1 to L3 are listed in Table 1. As it can be seen from the Table 1, the ratios of the ellipse axis radii, for example for the turn number T1a in individual tiers, are the same in spite of the increasing sizes of both ellipse radii of this turn in the higher tiers of the stimulation coil, so as to ensure that the enlargement of the individual tiers takes place at an angle of approx. 45 degrees. However, it is possible to make a stimulation coil in which these ratios will differ more or less.

Fig. 2 is a bottom view of the stimulation coils of Fig. 1, with the difference that the respective turns are distinguished here by different shades. The turns T1a to T6a and T1b to T6b are in the individual tiers L1 to L3. The turns T1a to T5a and T1b to T5b of the lower tier L1 are in light colour, in the medium gray are turns T1a to T6a and T1b to T6b of the middle (second) tier L2 and in the dark colour are the turns T1a to T6a and T1b to T6b of the upper tier L3.

**Table 1**

| | Tier I | Tier II | Tier III | Tier IV |
|---|---|---|---|---|
| Turn T1 | 0.55 | 0.55 | 0.55 | 0.55 |
| Turn T2 | 0.62 | 0.62 | 0.62 | 0.62 |
| Turn T3 | 0.69 | 0.69 | 0.69 | 0.69 |
| Turn T4 | 0.76 | 0.76 | 0.76 | 0.76 |
| Turn T5 | 0.83 | 0.83 | 0.83 | 0.83 |
| Turn T6 | - | 0.90 | 0.90 | 0.90 |

The intended focus of the stimulation coil 1 is located in the depth of about 2 to 3 cm from the bottom surface of the turns T1a to T5a and T1b to T5b of the winding conductor in the bottom tier L1 of the stimulation coil 1, i.e. in the tier L1, which is the closest to the application side 5 of the stimulation coil 1, which is used for simulation, and this under the centre of the stimulation coil 1 so that it approximately covers the motor cortex region of the brain.

In the central region 3 of the stimulation coil 1, the turns, situated in the individual tiers L1 to L3 are bent so that on the application side 5 this bending of the turns forms a 1 cm deep spherical depression 6 in the central region 3 of the stimulation coil 1.

The bending of the turns T1a to T5a and T1b to T5b in the tier L1 and of the turns T1a to T6a and T1b to T6b in the tiers L2 and L3 in the middle of the central region 3 of the stimulation coil 1 ensures the highest possible intensity in the central axis of the stimulation coil 1, which is required to be ensured.

The spherical depression 6 is formed so that the turns T1a to T5a and T1b to T5b in the tier L1 and the turns T1a to T6a and T1b to T6b in the tiers L2 and L3 of the stimulation coil 1 are bent respectively in the direction from the application side 5, so that forms a spherical depression 6 is formed in the central region 3 without affecting in any way the dimensions of the conductor of the individual turns lying in this region.

As it can be seen from the illustration of the stimulation coil, the turns T1a to T5a and T1b to T5b in the tier L1 and the turns T1a to T6a and T1b to T6b in the tiers L2 and L3 of both halves 2a, 2b of the winding in the individual tiers L1 to L3 in the central region 3 are made linearly and as close as possible to each other, i.e. without gaps between the individual turns, to ensure the highest possible concentration of conductors, and thus to produce the highest possible required magnetic field strength in the focus of the stimulation coil.

Fig. 3 shows the left half of the winding 2a, which has the width of the central region ½ A, but it has four tiers L1 to L4.

The spacing of the individual turns in the individual winding tiers in the refluent regions 4 of the stimulation coil 1 is advantageous due to the reduction of the local intensity of the magnetic field, what also increases the intensity in the central axis.

The loosening of the turns in the region axially distant from the central region 3 does not have such an effect, since the effect of increasing the loop area and diffusing of the intensity of the magnetic field is already prevailing there, so that it is not necessary for the individual turns to be loosen there.

Fig. 4 shows a side view of another embodiment of the stimulation coil 1 with four tiers L1 to L4.0 Due to the similarity of the design features to those of the stimulation coil 1 in Figs. 1 to 2, the coil will not be described in more detail here again. All tiers L1 to L4 of the embodiment of the stimulation coil 1 in Fig. 4 are tilted by an angle α of about 10 degrees.

This figure also shows the upward arching of the central region 3, formed by the turn portions T1a to T6a and T1b to T6b of the last tier L4 of both halves 2a, 2b of the winding upwards, which arching is caused by formation of a spherical recess 6 on the side to be used for therapy.

This tilt of both halves 2 and 2b of all tiers L1 to L4 does not affect the intensity in the central axis of the stimulation coil 1 significantly, but it reduces the undesired effects of the lateral lobes, what would otherwise result in undesired unpleasant stimulation of the head muscles.

It is obvious to the person skilled in the art that the tilting of the tiers L1 to L4 shown in Fig. 4 is fully applicable also to the embodiment with three tiers L1 to L3 shown in Figs. 1 and 2.

Fig. 5 then shows an embodiment of the turns T1a to T5a and T1b to T5b of the bottom tier L1 of the stimulation coil 1, wherein the turns T1a to T5a in the left half of the winding 2a are shown in light colour, and the turns T1b to T5b in the right half of the winding 2b are shown in dark colour.

It is obvious to a person skilled in the art that the described features of the stimulation coils are mutually combinable and that their application does not depend neither on the number of the coil tiers nor on the number of turns in individual tiers.

Furthermore, the above specified dimensions and characteristics of embodiments of the stimulating coil should be understood only as exemplary, and they are not intended to be limiting in any case.

For example, other embodiments are possible to be implemented, in which the stimulation coil will be, for example, only of a half of the above dimension, but it may also be a double of that dimension. The stimulation coil can be provided with a different number of turns in individual tiers and a different number of tiers.

This means, of course, that the dimensions of the stimulation coil can vary both within the scope of the specified dimensions and outside of them.

The examples of the possible embodiments of the stimulating coil of the present invention certainly are not intended to limit the scope of this invention only to those embodiments, nor to the dimensions described and similar data.

Therefore, the subject matter of the invention is limited only by the wording of the claims.

### List of Reference Signs:

- 1: Stimulation coil
- 2a: Left half of stimulation coil
- 2b: Right half of stimulation coil
- 3: Central region
- 4: Refluent region
- 5: Application side
- 6: Spherical depression
- L1-L4: Tiers of stimulation coil
- T1a - T6a: Turns of a conductor in a tier of the left half of stimulation coil
- T1b - T6b: Turns of a conductor in a tier of the right half of stimulation coil

## Claims

1. A magnetic stimulation coil (1) consisting of two symmetrical halves, a left half (2a) and a right half (2b), wherein both halves (2a, 2b) are arranged next to each other and in side-by-side relation, the winding of each half (2a, 2b) being formed by a set of turns (T) made of a conductor, wherein said stimulation coil (1) has an application side (5), and wherein winding of each half (2a, 2b) is formed by at least three tiers (L1 to Ln) of turns, each tier (L1 to Ln) being formed by a group of the turns (T1 to Tm), wherein the tiers (L1 to Ln) are arranged one on another, "n" corresponding to number of tiers and "m" corresponding to number of turns in a given tier, the turns (T1 to Tm) of the conductor are arranged in a shape of an ellipse in each tier (L1 to Ln) of the winding, being arranged into a central region (3) and into a refluent region (4); in the central region the turns are arranged close to each other without any substantial separating gaps between them to ensure their greatest possible density and efficiency to induce magnetic field in a focus situated under the centre of the central region, wherein in the refluent region the turns are spaced apart from each other by a gap having the size of at least a half of the smallest of the dimensions of the conductor cross-section at least in the region of both vertices of the ellipse; at least the outer turn of the conductor of each tier (L1 to Ln) forming an ellipse having a ratio of radii between 0.3 - 1.0; all the tiers (L1 to Ln) in each half (2a, 2b) are connected in series and have the same direction of the conductor rotation, wherein said direction of conductor rotation is opposite in each half (2a, 2b) to provide the same direction of current passing through the turns (T1 to Tm) in the central region (3) in both halves (2a, 2b); and the turns (T1 to Tm) of the conductor in the individual tiers (L1 to Ln) widen in the region of the vertices of the ellipse with regard to the turns of the conductor in the previous tier, in a direction from the application side (5) to ensure directing of the magnetic field towards the focus.

2. The stimulation coil according to claim 1, wherein in the central region (3) the turns of the individual tiers (L1 to Ln) of the winding are bent to form a substantially spherical depression (6) on the application side (5) having the greatest depth in the range of 0.5 to 1.5 cm.

3. The stimulation coil according to claim 2, wherein the spherical depression (6) is having the maximum depth of about 1 cm.

4. The stimulation coil according to claim 1, wherein in the refluent region (4) of each winding half (2a, 2b) the individual conductor turns (T1 to Tm) in each tier (L1 to Ln) are spaced apart from each other to at most three diameters of this conductor at least in the area of both vertices of the ellipse.

5. The stimulation coil according to anyone of claims 1 to 4, wherein both winding halves (2a, 2b) are inclined at an angle of 5 to 20 degrees in the direction away from the central region in the direction away from the application side (5).

6. The stimulation coil according to claim 5, wherein both halves of the winding are inclined in the direction away from the central region by an angle of about 10 degrees in the direction away from the application side.

7. The stimulation coil according to any one of claims 1 to 6, wherein the ratio of the radii is about 0.6 to 0.7.

8. The stimulation coil according to any one of claims 1 to 7, wherein each half of the winding has 4 tiers.

9. The stimulation coil according to any one of claims 1 to 8, wherein the widening of the individual turns of every further tier is provided in a region of the vertices of the ellipse from the stimulation side (5) so that the outside points of their circumferences form a line having an angle of approximately 45 degrees with the plane of stimulation.

## Patentansprüche

1. Eine magnetische Stimulationsspule (1), bestehend aus zwei symmetrischen Hälften, einer linken Hälfte (2a) und einer rechten Hälfte (2b), wobei beide Hälften (2a, 2b) nebeneinander und in Seiten-an-Seiten-Beziehung angeordnet sind, die Wicklung jeder Hälfte (2a, 2b) durch eine Anzahl von Windungen (T) aus einem Leiter gebildet ist, wobei die Stimulationsspule (1) eine Anwendungsseite (5) aufweist, und wobei die Wicklung jeder Hälfte (2a, 2b) aus mindestens drei Lagen (L1 bis Ln) von Windungen besteht, wobei jede Lage (L1 bis Ln) durch eine Gruppe von Windungen (T1 bis Tm) gebildet ist, wobei die Lagen (L1 bis Ln) übereinander angeordnet sind, "n" der Anzahl der Lagen und "m" der Anzahl der Windungen in einer gegebenen Lage entspricht, die Windungen (T1 bis Tm) des Leiters in jeder Lage (L1 bis Ln) der Wicklung in Form einer Ellipse angeordnet sind, wobei sie in einem Zentralbereich (3) und in einem Rückflussbereich (4) angeordnet sind; im Zentralbereich sind die Windungen dicht beieinander ohne wesentliche Trennabstände angeordnet, um ihre größtmögliche Dichte und Effizienz zu gewährleisten, um ein Magnetfeld in einem Fokus unter dem Zentrum des Zentralbereichs zu induzieren, wobei im Rückflussbereich die Windungen durch einen Spalt voneinander beabstandet sind, der mindestens die Hälfte der kleinsten Abmessung des Leiterquerschnitts beträgt, zumindest in dem Bereich beider Scheitelpunkte der Ellipse, wobei zumindest die äußere Windung des Leiters jeder Lage (L1 bis Ln) ein Ellipsenverhältnis der Radien zwischen 0,3 - 1,0 bildet; alle Lagen (L1 bis Ln) in jeder Hälfte (2a, 2b) sind in Reihe geschaltet und haben die gleiche Drehrichtung des Leiters, wobei die Drehrichtung des Leiters in jeder Hälfte (2a, 2b) entgegengesetzt ist, um dieselbe Stromrichtung durch die Windungen (T1 bis Tm) im Zentralbereich (3) in beiden Hälften (2a, 2b) bereitzustellen; und die Windungen (T1 bis Tm) des Leiters in den einzelnen Lagen (L1 bis Ln) weiten sich im Bereich der Scheitelpunkte der Ellipse im Vergleich zu den Windungen des Leiters in der vorherigen Lage in Richtung von der Anwendungsseite (5), um die Ausrichtung des Magnetfelds auf den Fokus sicherzustellen.

2. Stimulationsspule nach Anspruch 1, wobei im Zentralbereich (3) die Windungen der einzelnen Lagen (L1 bis Ln) der Wicklung so gebogen sind, dass auf der Anwendungsseite (5) eine im Wesentlichen sphärische Vertiefung (6) mit einer größten Tiefe im Bereich von 0,5 bis 1,5 cm gebildet ist.

3. Stimulationsspule nach Anspruch 2, wobei die sphärische Vertiefung (6) eine maximale Tiefe von etwa 1 cm aufweist.

4. Stimulationsspule nach Anspruch 1, wobei im Rückflussbereich (4) jeder Wicklungshälfte (2a, 2b) die einzelnen Leiterwindungen (T1 bis Tm) in jeder Lage (L1 bis Ln) voneinander in einem Abstand von höchstens dem Dreifachen des Durchmessers dieses Leiters beabstandet sind, zumindest im Bereich beider Scheitelpunkte der Ellipse.

5. Stimulationsspule nach einem der Ansprüche 1 bis 4, wobei beide Wicklungshälften (2a, 2b) in einem Winkel von 5 bis 20 Grad in Richtung weg vom Zentralbereich in Richtung weg von der Anwendungsseite (5) geneigt sind.

6. Stimulationsspule nach Anspruch 5, wobei beide Hälften der Wicklung in Richtung weg vom Zentralbereich um einen Winkel von etwa 10 Grad in Richtung weg von der Anwendungsseite geneigt sind.

7. Stimulationsspule nach einem der Ansprüche 1 bis 6, wobei das Verhältnis der Radien etwa 0,6 bis 0,7 beträgt.

8. Stimulationsspule nach einem der Ansprüche 1 bis 7, wobei jede Hälfte der Wicklung 4 Lagen aufweist.

9. Stimulationsspule nach einem der Ansprüche 1 bis 8, wobei die Erweiterung der einzelnen Windungen jeder weiteren Lage in einem Bereich der Scheitelpunkte der Ellipse von der Stimulationsseite (5) derart vorgesehen ist, dass die Außenpunkte ihres Umfangs eine Linie bilden, die einen Winkel von ungefähr 45 Grad mit der Stimulationsebene bildet.

## Revendications

1. Bobine de stimulation magnétique (1) consistant en deux moitiés symétriques, une moitié gauche (2a) et une moitié droite (2b), dans laquelle les deux moitiés (2a, 2b) sont disposées l'une près de l'autre et côte à côte, l'enroulement de chaque moitié (2a, 2b) étant formé par un ensemble de spires (T) constituées d'un conducteur, ladite bobine de stimulation (1) ayant un côté d'application (5), et l'enroulement de chaque moitié (2a, 2b) étant formé par au moins trois niveaux (L1 à Ln) de spires, chaque niveau (L1 à Ln) étant formé par un groupe de spires (T1 à Tm), les niveaux (L1 à Ln) étant disposés les uns sur les autres, "n" correspondant au nombre de niveaux et "m" correspondant au nombre de spires dans un niveau donné, les spires (T1 à Tm) du conducteur étant disposées dans une forme d'une ellipse dans chaque niveau (L1 à Ln) de l'enroulement, étant disposées dans une région centrale (3) et dans une région de reflux (4) ; dans la région centrale, les spires étant disposées les unes près des autres sans espaces de séparation substantiels entre elles, afin d'assurer leur densité et leur efficacité les plus élevées possibles pour induire un champ magnétique dans un foyer situé sous le centre de la région centrale, dans la région de reflux, les spires étant espacées les unes des autres par un espace ayant la taille d'au moins une moitié de la plus petite des dimensions de la section transversale du conducteur au moins dans la région des deux sommets de l'ellipse ; au moins la spire externe du conducteur de chaque niveau (L1 à Ln) formant une ellipse dont le rapport des rayons est compris entre 0,3 - 1,0 ; tous les niveaux (L1 à Ln) dans chaque moitié (2a, 2b) étant connectés en série et ayant le même sens de la rotation du conducteur, ledit sens de rotation du conducteur étant opposé dans chaque moitié (2a, 2b) pour assurer le même sens du courant circulant à travers les spires (T1 à Tm) dans la région centrale (3) dans les deux moitiés (2a, 2b) ; et les spires (T1 à Tm) du conducteur dans les différents niveaux (L1 à Ln) s'élargissant dans la région des sommets de l'ellipse par rapport aux spires du conducteur dans le niveau précédent, dans un sens partant du côté d'application (5) pour assurer le sens du champ magnétique vers le foyer.

2. Bobine de stimulation selon la revendication 1, dans laquelle, dans la région centrale (3), les spires des différents niveaux (L1 à Ln) de l'enroulement sont courbées pour former une dépression (6) sensiblement sphérique sur le côté d'application (5) ayant la plus grande profondeur dans la plage de 0,5 à 1,5 cm.

3. Bobine de stimulation selon la revendication 2, dans laquelle la dépression sphérique (6) a la profondeur maximale d'environ 1 cm.

4. Bobine de stimulation selon la revendication 1, dans laquelle, dans la région de reflux (4) de chaque moitié d'enroulement (2a, 2b), les spires de conducteur individuelles (T1 à Tm) dans chaque niveau (L1 à Ln) sont espacées les unes des autres d'au plus trois diamètres de ce conducteur au moins dans la zone des deux sommets de l'ellipse.

5. Bobine de stimulation selon l'une quelconque des revendications 1 à 4, dans laquelle les deux moitiés d'enroulement (2a, 2b) sont inclinées à un angle de 5 à 20 degrés dans la direction opposée à la région centrale dans la direction opposée au côté d'application (5).

6. Bobine de stimulation selon la revendication 5, dans laquelle les deux moitiés de l'enroulement sont inclinées dans la direction opposée à la région centrale d'un angle d'environ 10 degrés dans la direction opposée au côté d'application.

7. Bobine de stimulation selon l'une quelconque des revendications 1 à 6, dans laquelle le rapport des rayons est d'environ 0,6 à 0,7.

8. Bobine de stimulation selon l'une quelconque des revendications 1 à 7, dans laquelle chaque moitié de l'enroulement a 4 niveaux.

9. Bobine de stimulation selon l'une quelconque des revendications 1 à 8, dans laquelle l'élargissement des spires individuelles de chaque autre niveau est assuré dans une région des sommets de l'ellipse à partir du côté de stimulation (5) de telle sorte que les points extérieurs de leurs circonférences forment une ligne ayant un angle d'environ 45 degrés avec le plan de stimulation.
